(19) Europäisches Patentamt
European Patent Office
Office européen des brevets

(11) Veröffentlichungsnummer: **0 389 648 B1**

(12) **EUROPÄISCHE PATENTSCHRIFT**

(45) Veröffentlichungstag der Patentschrift: **14.06.95**

(51) Int. Cl.[6]: **C11D 3/00**, C11D 1/94, A61K 7/50, A61K 7/40

(21) Anmeldenummer: **89105348.0**

(22) Anmeldetag: **25.03.89**

# (54) Mittel zur Händedekontaminierung und/oder -desinfektion, anwendbar durch einen Seifenspender.

(43) Veröffentlichungstag der Anmeldung:
**03.10.90 Patentblatt 90/40**

(45) Bekanntmachung des Hinweises auf die Patenterteilung:
**14.06.95 Patentblatt 95/24**

(84) Benannte Vertragsstaaten:
**AT BE CH DE ES FR GB GR IT LI LU NL SE**

(56) Entgegenhaltungen:
**EP-A- 0 024 031**
**DE-A- 1 962 238**
**US-A- 4 668 422**
**EP-A- 0 247 832**
**DE-A- 2 742 007**
**US-E- 32 300**

(73) Patentinhaber: **CWF-CHEMIE-FRANKFURT GmbH**
**Eichenheeg 18/20**
**D-63477 Maintal (DE)**

(72) Erfinder: **Uhlig, Bernd G.**
**Fichardstrasse 5**
**D-6000 Frankfurt/Main 1 (DE)**

(74) Vertreter: **Müller-Wolff, Thomas et al**
**HARWARDT NEUMANN,**
**Patent- und Rechtsanwälte,**
**Postfach 14 55**
**D-53704 Siegburg (DE)**

EP 0 389 648 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft Mittel zur Händedekontaminierung und/oder Desinfektion für die Dosierung durch Seifenspender, bestehend aus einer Kombination eines amphoteren Tensides, weiteren kationischen Tensiden und Netzmitteln und einem Rückfetter.

Ein Mittel der eingangs genannten Art ist aus der EP-A-0024031 bekannt. Aus der EP-A-0247832 ist ferner eine Reinigungslösung bekannt, die aus einem amphoteren Tensid und bestimmten kationischen und anionischen Tensiden besteht.

Ferner sind Reinigungsmittel bekannt, die zwar eine ausreichende Rückfettung besitzen, jedoch ist die Zeit für eine ausreichende Keimreduzierung entsprechend der 6. Liste der Deutschen Gesellschaft für die Hygiene und Mikrobiologie zu lang. Die Anforderungen an die Händedesinfektion sehen vor, daß eine Reduzierung der Keime um 3-log-Stufen, d.h. um 99,7 % erreicht wird, wobei eine Benetzungswilligkeit, etwa adäquat der der Alkohole, vorausgesetzt ist.

Bekannte Mittel für die Händedekontaminierung, die diese Anforderungen erfüllen, enthalten niedermolekulare Alkohole (Äthanol, N-Propanol, Isopropanol) sowie Jod, die zur Stabilisierung komplex gebunden sind. Amphotere und kationische Tenside werden zwar ebenfalls verwendet, jedoch nur in Kombination mit Alkoholen oder Jodoforen.

Diese Mittel haben jedoch eine Reihe gravierender Nachteile , insbesondere die,

- daß sie die Haut durch die stark entfettenden Alkohole und die nicht gegebene Möglichkeit einer mit der Desinfektion verbundenen Rückfettung stark angreifen;
- daß ihre Verwendbarkeit in Seifenspendern und damit die Unterbindung weiterer Keimbelastung als Folge der Aggressivität praktisch ausschließen. Dies gilt insbesondere für die Verwendung von Seifenspendern aus Kunststoff, da der Angriff durch Jodophore und/oder Alkohole durch Migration und Quellung der Kunststoffe die Mechanik der Behälter sehr schnell unbrauchbar macht.
- daß die Verwendbarkeit dieser in der Regel gelartigen Seifenlösung nicht deren Weiterbildung zur Abgabe in Schaumform, die nur ein Minimum von Wasser erfordert, und damit die zusätzliche Zuführung von Keimen aus dem Leitungswasser einschränkt, nicht ermöglicht, da die alkoholhaltigen Seifen schaumzerstörend wirken.

Aus dieser nicht vollständigen Aufzählung der Nachteile des eingangs genannten Standes der Technik resultiert die Aufgabenstellung, ein Mittel zu nennen, das bei gleicher Desinfektions- und Dekontaminierungswirkung nicht hautaggressiv ist, und das die Wahl des Werkstoffes für die Seifenspender praktisch nicht einschränkt.

Diese Aufgabe wird erfindungsgemäß durch ein Mittel mit der im Hauptanspruch angegebenen Zusammensetzung gelöst.

Mit diesem Mittel werden die Forderungen der Aufgabenstellung in vollem Umfang erfüllt.

Es ist eine weitestgehende Schonung der Haut durch Vermeidung der stark entfettenden Alkohole in Verbindung mit der Verwendung von Rückfettern gegeben.

Das Mittel kann durch normale Seifenspender dosiert werden, da der häufig beobachtete Angriff der Kunststoffe durch Jodophore und/oder Alkohole, d.h. durch Migration oder Quellung nicht möglich ist.

Desgleichen scheidet die Zerstörung von als Schaum aufbereiteten Mitteln aus, da die zerstörenden Alkohole fehlen.

Bevorzugte Ausführungsbeispiele des erfindungsgemäßen Mittels sind wie folgt zusammengesetzt:

a) 1 bis 8 Gewichtsteile eines amphoteren Tensides der Struktur

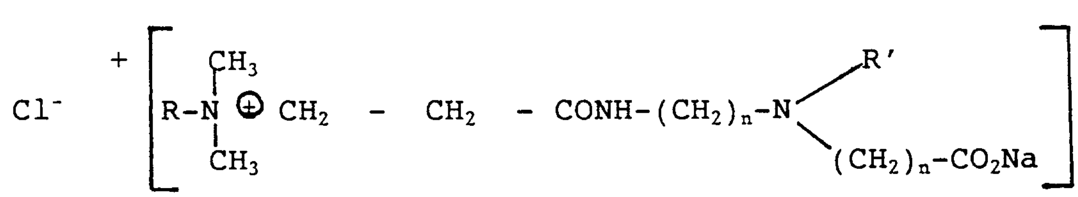

wobei

R = $C_{12}$ - $C_{18}$ - Alkyl

R' = $C_1$-$C_4$ -Alkyl oder $C_1$ bis $C_4$ Hydroxyalkyl und

n = 2 sind;

b) 2 bis 10 Gewichtsteile eines weiteren kationischen Tensides vom Benzalkoniumchlorid-Typ;

c) 0,5 bis 3 Gewichtsteile Sulfosuccinate ;

d) 1 bis 4 Gewichtsteile eines nichtionischen Rückfetters (Fettsäurealkylolamid).

Es wird folgendes Beispiel als Händedesinfektionsmittel genannt:

3 Tl. N[N'(N''-hydroxyethyl-N''-carboxyethyl) aminoethyl-essigsäureamido]N,N-dimethyl-N-laurylammoniumchlorid,
5 Tl. Benzalkoniumchlorid,
1 T. Lauryldimethylaminoxid
2 Tl. Kokosfettsäuremonoehtanolamid
89 Tl. Wasser
100 Tl. Flüssigseife

Nach einem Test **eines bekannten Institutes**, dessen sachliche Aussage vollinhaltlich nachstehend übernommen wird, sind folgende Ergebnisse bekannt:

" auftragsgemäß haben wir geprüft,ob die Flüssigseife keimtötende Eigenschaften gegenüber Bakterien, Hefen und Schimmelpilzen besitzt. Die Prüfung erfolgte durch einen Belastungstest.

Testkeime: Escherichia coli ATCC 15442 Staphylococcus aureus ATCC 6538 Bacillus subtilis ATCC 6051 Candida albicans ATCC 10231 Aspergillus niger ATCC 9642

Befund: Gegenüber allen Testkeimen bestehen gut ausgeprägte antimikrobielle Eigenschaften.

Zur näheren Prüfung dieser antimikrobiellen Eigenschaften wurden Untersuchungen nach den DGHM-Richtlinien für die Prüfung und Bewertung chemischer Desinfektionsverfahren durchgeführt.

1. Verdünnungstest

Testkeime : Escherichia coli, Pseudomonas aeruginosa, Staphylococcus aureus, Proteus mirabilis, Candida albicans.

Befund : Eine bakteriestatische und fungistatische Wirksamkeit besteht noch bei einer Konzentrationsstufe von ca. 25%. Da das Präparat unverdünnt eingesetzt wird, besteht eine sichere Wirksamkeit.

2. Suspensionsteste

a) qualitativ

Testkeime analog dem Verdünnungstest

Bei Einsatz von ca. $10^8$ Keimen/ml wird geprüft, in welcher Zeit die Testkeime abgetötet sind.

Ergebnisse:

| | | | |
|---|---|---|---|
| Testkeim | Escherichia coli | Pseudomonas aeruginosa | Staphyl. aureus |
| Zeit | 1,0 min. | 2,0 min. | 0,5 min. |
| Testkeim | Proteus mirabilis | Candida albicans | |
| Zeit | 2,0 min. | 1,0 min. | |

b) quantitativ

Testkeime : Staphylococcus aureus, Proteus mirabilis

Bei Einsatz von ca. $10^8$ Keimen/ml wird geprüft, welche Absterberaten in Abhängigkeit von der Einwirkzeit bei Gegenwart von Eiweiß (0,2% Rinderalbumin) und bei Abwesenheit von Eiweiß bestehen.

Ergebnisse:

| | | |
|---|---|---|
| Testkeime | : Staphyl. aureus mit Eiweiß | ohne Eiweiß |
| Einwirkzeit | : 0,5 \| 1 \| 2 \| 5 | 0,5 \| 1 \| 2 \| 5 min. |
| Absterberate in % | : 100----- | 100 - - - |

| | | |
|---|---|---|
| Testkeime | : Proteus mirabilis mit Eiweiß | ohne Eiweiß |
| Einwirkzeit | : 0,5 \| 1 \| 2 \| 5 | 0,5 \| 1 \| 2 \| 5 min. |
| Absterberate in % | : 99,6 99,9 100- | 99,7 99,9 100 - |

Bewertung: Das geprüfte Produkt besitzt gute antimikrobielle Eigenschaften und ist zur Händedekontamination grundsätzlich geeignet. Ein signifikanter Eiweißfehler besteht nicht.

Pf.-Nr. 8805ML30531 + 8807ML37577 "

Das in einem Schaumseifenspender einsetzbare Mittel sieht vor, daß
4 Tl. N[N'(N''-hydroxymethyl-N''-carboxymethyl) aminoethyl-essigsäureamido-]-N,N-dimethyl-N-cocosammoniumchlorid
4 Tl. Lauryldimethylammoniummethosulfat
1 Tl. Diisooctylfosuccinat
2 Tl. Laurinsäuremonoethanolamid
verwendet werden.

Diese Mischung ergibt im Schaumseifenspender einen leichten, cremig-feinblasigen Schaum, der, nach Reinigung der Hände, ein seifig glattes Hautgefühl hinterläßt.

## Patentansprüche

**1.** Mittel zur Händedekontamination und/oder Desinfektion für die Dosierung durch Seifenspender, bestehend aus einer Kombination eines amphoteren Tensides, weiteren kationischen Tensiden und Netzmitteln und einem Rückfetter,
gekennzeichnet durch eine der folgenden Zusammensetzungen:
a) 1 bis 8 Gewichtsteile eines amphoteren Tensides der Struktur N[N'(N''-(hydroxy)alkyl-N''-carboxyalkyl) aminoethylessigsäureamido]-N,N-dimethyl-N-alkylammoniumchlorid und
b) 2 bis 10 Gewichtsteile eines weiteren kationischen Tensides vom Benzalkoniumchlorid-Typ und
c) 0,5 bis 3 Gewichtsteile eines mit kationischen Tensiden verträglichen Netzmittels, wie Sulfosuccinaten, Naphthalinsulfonat oder Aminoxyden und
d) 1 bis 4 Gewichtsteile eines nichtionogenen Rückfetters oder
a') 4 Gewichtsteile N[N'(N''-hydroxymethyl-N''-carboxy methyl) aminoethyl-essigsäureamido]-N,N-dimethyl-N-cocosammoniumchlorid,
b') 4 Gewichtsteile Lauryldimethylammoniummethosulfat
c') 1 Gewichtsteile Diisooctylfosuccinat
d') 2 Gewichtsteile Laurinsäuremonoethanolamid,
wobei die Mittel gegebenenfalls Wasser enthalten.

**2.** Mittel nach Anspruch 1,
dadurch gekennzeichnet,
daß als Sulfosuccinat (c) Diisooctylsulfosuccinat verwendet wird.

**3.** Mittel nach Anspruch 1,
dadurch gekennzeichnet,

daß als Naphthalinsulfonat (c) Natriumdisooctylnaphthalinsulfonat verwendet wird.

**4.** Mittel nach Anspruch 1,
dadurch gekennzeichnet,
daß als Aminoxyd (c) Lauryldimethylaminoxid eingesetzt wird.

**5.** Mittel nach Anspruch 1,
dadurch gekennzeichnet,
daß als nichtionogener Rückfetter (d) Fettsäurealkylolamid eingesetzt wird.

**6.** Mittel nach Anspruch 1,
dadurch gekennzeichnet,
daß als Rückfetter (d) ethoxiliertes Partialglycerid eingesetzt wird.

## Claims

**1.** An agent for decontaminating hands and/or for disinfecting purposes, such agent being metered by soap dispensers and consisting of a combination of amphoteric surfactants, further cationic surfactants and wetting agents and a re-greasing agent,
characterised by one of the following combinations:
a) 1 to 8 parts by weight of an amphoteric surfactant of the structure N[N'(N''-(hydroxy)alkyl-N''-carboxyalkyl) aminoethyl acetic acid amido]-N,N-dimethyl-N-alkylammonium chloride and
b) 2 to 10 parts by weight of a further cationic surfactant of the benzalkonium chloride type and
c) 0.5 to 3 parts by weight of a wetting agent compatible with cationic surfactants, such as sulfosuccinates, naphthalen sulphonate or amine oxides and
d) 1 to 4 parts by weight of a non-ionogenic re-greasing agent or
a') 4 parts by weight of N[N'(N''-hydroxymethyl-N''-carboxy methyl) aminoethyl acetic acid amido]-N,N-dimethyl-N-cocosammonium chloride,
b') 4 parts by weight of lauryldimethylammoniummethosulphate,
c') 1 part by weight of diisooctylfosuccinate,
d') 2 parts by weight of lauric acid monoethanolamide,
with said agents optionally containing water.

**2.** An agent according to claim 1,
characterised in
that sulfosuccinate (c) is used in the form of diisooctylsulfosuccinate.

**3.** An agent according to claim 1,
characterised in
that naphthalin sulphonate (c) is used in the form of sodium disooctylnaphthalinsulphonate.

**4.** An agent according to claim 1,
characterised in
that amine oxide (c) is used in the form of lauryldimethyl amine oxide.

**5.** An agent according to claim 1,
characterised in
that the non-iogenic re-greasing agent (d) is used in the form of fatty acid alkylolamide.

**6.** An agent according to claim 1,
characterised in
that the re-greasing agent (d) is used in the form of ethoxilised partial glyceride.

## Revendications

**1.** Agent permettant la décontamination et/ou la désinfection des mains par l'intermédiaire d'un distributeur-doseur de savon, agent constitué par une combinaison comportant un agent tensioactif amphotère, des agents supplémentaires tensioactifs et mouillants qui sont cationiques et d'un agent dégraissant,

caractérisé par l'une des compositions suivantes:

a) de 1 à 8 parties en poids d'un agent tensioactif amphotère de structure N(N' de l'amide de l'acide (N''-(hydroxy)alkyl-N''-carboxyalkyl) aminoéthyl-acétique] - chlorure de N,N-diméthyl-N-alkylammonium et

b) de 2 à 10 parties en poids d'un agent supplémentaire tensioactif cationique du type chlorure de benzalconium et

c) de 0,5 à 3 parties en poids d'un agent dégraissant compatible avec les agents tensioactifs cationiques, tel que les sulfosuccinates, les naphtalinsulfonates ou les aminoxydes et

d) de 1 à 4 parties en poids d'un agent dégraissant non ionogène ou

a') 4 parties en poids de N[N' de l'amide de l'acide (N''-(hydroxy)méthyl-N''-carboxyméthyl) aminoéthyl-acétique] - chlorure de N,N-diméthyl-N-cocosammonium,

b') 4 parties en poids d'éthosulfate de lauryldiméthyl-ammonium,

c') 1 partie en poids de diisooctyl-sulfosuccinate,

d') 4 parties en poids de monoéthanolamide d'acide laurique, où les agents contiennent le cas échéant de l'eau.

**2.** Agent selon la revendication 1,
caractérisé en ce que,
on emploie comme sulfosuccinate (c) du diisooctyl-sulfosuccinate.

**3.** Agent selon la revendication 1,
caractérisé en ce que,
on emploie comme naphthalinsulfonate (c) du diisooctyl-naphthalinsulfonate de sodium.

**4.** Agent selon la revendication 1,
caractérisé en ce que,
on emploie comme aminoxyde (c) de l'aminoxyde de lauryl-diméthyle.

**5.** Agent selon la revendication 1,
caractérisé en ce que,
on emploie comme agent dégraissant non ionogène (d) de l'alkylolamide d'acide aliphatique.

**6.** Agent selon la revendication 1,
caractérisé en ce que,
on emploie comme agent dégraissant (d) une glycéride partiellement éthoxylée.